(19) Europäisches Patentamt / European Patent Office / Office européen des brevets

(11) **EP 3 207 397 B1**

(12) **FASCICULE DE BREVET EUROPEEN**

(45) Date de publication et mention de la délivrance du brevet:
**22.06.2022 Bulletin 2022/25**

(21) Numéro de dépôt: **15787262.3**

(22) Date de dépôt: **09.10.2015**

(51) Classification Internationale des Brevets (IPC):
**G01R 33/563** (2006.01)  **A61B 6/04** (2006.01)
**A61B 6/00** (2006.01)  **A61B 5/026** (2006.01)
**A61B 5/0275** (2006.01)  **A61B 5/055** (2006.01)
**G01R 33/56** (2006.01)

(52) Classification Coopérative des Brevets (CPC):
**G01R 33/56366; A61B 5/0263; A61B 5/0275;
A61B 5/055; A61B 6/481; A61B 6/486;
A61B 6/507; A61B 6/5217; G01R 33/5601;
G01R 33/56383;** A61B 6/0487

(86) Numéro de dépôt international:
**PCT/FR2015/052728**

(87) Numéro de publication internationale:
**WO 2016/059329 (21.04.2016 Gazette 2016/16)**

(54) **SYSTÈME ET PROCÉDÉ POUR ESTIMER UNE QUANTITÉ D'INTÉRÊT D'UN SYSTÈME DYNAMIQUE ARTÈRE/TISSU/VEINE**

SYSTEM UND VERFAHREN ZUR SCHÄTZUNG EINER RELEVANTEN MENGE EINES DYNAMISCHEN ARTERIEN-/GEWEBE-/VENENSYSTEMS

SYSTEM AND METHOD FOR ESTIMATING AN AMOUNT OF INTEREST OF A DYNAMIC ARTERY/TISSUE/VEIN SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.10.2014 FR 1459822**

(43) Date de publication de la demande:
**23.08.2017 Bulletin 2017/34**

(73) Titulaire: **Olea Medical**
**13600 La Ciotat (FR)**

(72) Inventeurs:
• DJERIDANE, Fayçal
  13004 Marseille (FR)
• BOUTELIER, Timothé
  13600 La Ciotat (FR)

(74) Mandataire: **Brun, Philippe Alexandre Georges**
**MED'iNVENT CONSULTING**
**Espace Mistral - Bât.A**
**297 avenue du Mistral**
**ZI ATHELIA IV**
**13705 La Ciotat Cedex (FR)**

(56) Documents cités:
FR-A1- 2 979 453      US-A1- 2008 114 234
US-A1- 2011 110 488      US-A1- 2013 123 611
US-B1- 6 628 743

• ANDERSON JUSTIN D ET AL: "1042 Multi-modality magnetic resonance demonstrates factors critical to functional capacity in peripheral arterial disease", JOURNAL OF CARDIOVASCULAR MAGNETIC RESONANCE, BIOMED CENTRAL LTD, LONDON UK, vol. 10, no. Suppl 1, 22 octobre 2008 (2008-10-22), page A167, XP021044149, ISSN: 1532-429X, DOI: 10.1186/1532-429X-10-S1-A167

- Lin S ET AL: "IRM CORPS ENTIER EN HAUTE RESOLUTION AVEC INJECTION DYNAMIQUE DE GADOLINIUM DANS LES SYNDROMES LYMPHOPROLIFERATIFS", Journal de Radiologie, vol. 88, no. 10, 1 October 2007 (2007-10-01), page 1445, XP55890583,
- Luciani A ET AL: "HEMATOLOGIE: APPLICATIONS IRM CORPS ENTIER", Journal de Radiologie, vol. 90, no. 10, 1 October 2009 (2009-10-01), page 1338, XP55890581,

**Description**

L'invention concerne une unité de traitement, un système , un produit programme d'ordinateur et un procédé pour estimer une quantité d'intérêt à partir de données de perfusion résultant de l'acquisition d'une pluralité de volumes ou stations d'un patient. L'invention se distingue notamment des procédés connus par sa précision et par une grande cohérence des paramètres estimés en lien avec une pluralité de stations, notamment pour étudier des organes.

L'invention s'appuie notamment sur des techniques d'imagerie de Perfusion par Résonance Magnétique (Perfusion Weighted Magnetic Resonance Imaging - PW-MRI - selon une terminologie anglo-saxonne). Ces techniques permettent d'obtenir rapidement des informations précieuses sur l'hémodynamique d'organes présents chez les êtres humains ou les animaux. Ces informations sont particulièrement cruciales pour un praticien cherchant à établir un diagnostic et à prendre une décision thérapeutique dans le traitement de pathologies.

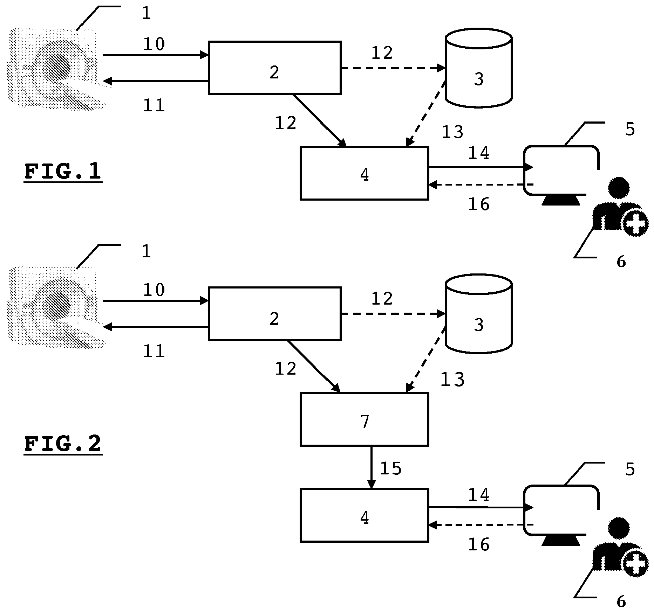

Pour mettre en œuvre de telles techniques, un appareil d'imagerie 1 par Résonance Magnétique Nucléaire , tel que celui illustré à titre d'exemple non limitatif par les figures 1 et 2, est en principe utilisé. Celui-ci peut délivrer une pluralité de séquences d'images numériques 12 d'une ou plusieurs parties du corps, à titre d'exemples non limitatifs, le cerveau, le cœur, les poumons, etc. Ledit appareil applique pour cela une combinaison d'ondes électromagnétiques à haute fréquence sur la partie du corps considérée et mesure le signal réémis par certains atomes, tels qu'à titre d'exemple non limitatif, l'hydrogène pour l'imagerie par Résonance Magnétique Nucléaire. L'appareil permet ainsi de déterminer la composition chimique et donc la nature des tissus biologiques en chaque volume élémentaire, tel qu'un voxel, du volume imagé. L'appareil 1 d'imagerie par Résonance Magnétique Nucléaire est commandé à l'aide d'une console 2. Un utilisateur peut ainsi choisir des paramètres 11 pour piloter l'appareil 1. A partir d'informations 10 produites par l'appareil 1, on obtient une pluralité de séquences d'images numériques 12 d'une partie d'un corps d'un humain ou d'un animal. L'appareil 1 d'imagerie par Résonance Magnétique Nucléaire peut être éventuellement inclus au sein d'un système d'analyse d'imagerie.

En variante, ledit appareil 1 peut être dissocié du système d'analyse. Selon cette variante, les séquences d'images 12 peuvent optionnellement être stockées au sein d'un serveur 3 et constituer un dossier médical 13 d'un patient. Un tel dossier 13 peut comprendre des images de différents types, telles que des images de perfusion ou de diffusion. Les séquences d'images 12 sont analysées au moyen d'une unité de traitement 4 dédiée. Ladite unité de traitement comporte des moyens pour communiquer avec le monde extérieur pour recueillir les images. Lesdits moyens pour communiquer permettent en outre à l'unité de traitement 4 de délivrer in fine, par l'intermédiaire de moyens de restitution 5 proposant un rendu graphique, sonore ou autre, à un praticien 6 ou à un chercheur, une estimation d'une ou plusieurs quantités d'intérêt, éventuellement formatées sous la forme d'un contenu, telles que des paramètres hémodynamiques 14, à partir des images numériques 12, avantageusement des images de perfusion, au moyen d'une interface homme-machine adaptée. L'utilisateur 6, avantageusement praticien, du système d'analyse peut ainsi confirmer ou infirmer un diagnostic, décider d'une action thérapeutique qu'il jugera adéquate, approfondir des travaux de recherche... Optionnellement, cet utilisateur 6 peut paramétrer le fonctionnement de l'unité de traitement 4 ou des moyens de restitution 5, au moyen de paramètres 16. Par exemple, il peut ainsi définir des seuils d'affichage ou choisir les paramètres estimés qu'il souhaite visualiser. Il existe une variante, décrite en liaison avec la figure 2, pour laquelle un système d'imagerie, tel que décrit précédemment, comporte en outre une unité de prétraitement 7 pour analyser les séquences d'images, pour en déduire des signaux expérimentaux 15 (de perfusion ou de perméabilité) et les délivrer à l'unité de traitement 4 qui est ainsi déchargée de cette tâche. Par ailleurs, pour réaliser une estimation de paramètres hémodynamiques ou plus généralement une quantité d'intérêt, l'unité de traitement 4 comporte généralement des moyens de traitement, tels qu'un calculateur, pour mettre en œuvre un procédé d'estimation sous la forme d'un programme préalablement chargé dans des moyens de mémorisation coopérant avec lesdits moyens de traitement.

Des images de perfusion par Résonance Magnétique Nucléaire sont obtenues en injectant un agent de contraste, par exemple un sel de gadolinium pour l'Imagerie par Résonance Magnétique, par voie intraveineuse et en enregistrant son bol au cours du temps au niveau de chaque voxel de l'image, par des acquisitions à intervalles réguliers. De telles images permettent notamment de caractériser la circulation sanguine dans le tissu d'un organe donné, en suivant l'évolution de la concentration de l'agent de contraste dans chaque voxel du volume acquis en fonction du temps. Au sens de l'invention et dans tout le document, on entend par « voxel » (contraction anglo-saxonne du terme « volumetric pixel »), un volume élémentaire permettant de mesurer la définition d'une image numérique matricielle en trois dimensions. Un tel voxel peut être encore considéré comme un pixel en trois dimensions. Dans tous les cas, un tel voxel est un parallélépipède rectangle dont la surface fermée est constituée par ses six faces.

Par souci de concision, nous omettrons les indices x,y,z pour identifier des voxels. Par exemple, au lieu de noter $S_{x,y,z}(t)$ le signal pour un voxel de coordonnées x,y,z en fonction du temps, nous le noterons simplement $S(t)$. Il est entendu que les opérations et les calculs décrits dans la suite sont généralement effectués pour chaque voxel d'intérêt, de sorte à obtenir au final des images ou des cartes représentatives d'une quantité d'intérêt, plus particulièrement des paramètres hémodynamiques, que l'on cherche à estimer.

Un modèle standard permet de relier l'intensité des signaux $S(t)$ mesurée au cours du temps $t$ à la concentration $C(t)$ dudit agent de contraste.

En Imagerie de Perfusion par Résonance Magnétique Nucléaire, il existe une relation exponentielle $S(t) = S_0 \cdot e^{-k \cdot TE \cdot C(t)}$. Dans les deux cas, $S_0$ représente l'intensité moyenne du signal avant l'arrivée de l'agent de contraste. En ce qui concerne l'imagerie par Résonance Magnétique Nucléaire, $k$ est une constante dépendant de la relation entre la susceptibilité paramagnétique et la concentration de l'agent de contraste dans le tissu et $TE$ est le temps d'écho (echo time selon une terminologie anglo-saxonne). La valeur de la constante k pour chaque voxel étant inconnue, celle-ci est fixée à une valeur arbitraire pour tous les voxels d'intérêt. On obtient ainsi des estimations relatives et non pas absolues. Ces informations relatives restent toutefois pertinentes puisque l'on est intéressé principalement par la variation relative de ces valeurs dans l'espace, en particulier entre les tissus sains et les tissus pathologiques.

La conservation de la masse de l'agent de contraste dans le volume de tissu contenu dans chaque voxel à chaque instant s'écrit

$$\frac{dC(t)}{dt} = BF \cdot \left[ C_a(t) - C_v(t) \right] . \quad C_a(t)$$

est la concentration de l'agent de contraste dans l'artère alimentant le volume de tissu (fonction d'entrée artérielle ou Arterial Input Fonction - AIF - selon une terminologie anglo-saxonne). $BF$ est le flux sanguin dans le volume de tissu (Blood Flow selon une terminologie anglo-saxonne) et $C_v(t)$ est la concentration de l'agent de contraste dans la veine drainant le volume de tissu (fonction de sortie veineuse ou Venous Output Function - VOF - selon une terminologie anglo-saxonne).

En supposant le système dynamique artère/tissu/veine linéaire et invariant dans le temps, on peut écrire $C_v(t) = C_a(t) \otimes h(t)$ où $h(t)$ est la réponse impulsionnelle du système et $\otimes$ désigne le produit de convolution. Une solution formelle de l'équation différentielle précédente avec condition initiale $C(t=0)=0$ s'écrit alors $C(t) = BF \cdot C_a(t) \otimes R(t)$ où $R(t)$ est la fonction des résidus (ou Residue Function selon une terminologie anglo-saxonne) définie par

$$R(t) = H(t) - \int_0^t h(\tau) d\tau$$

où $H$ est la fonction généralisée créneau de Heaviside.

Les paramètres hémodynamiques tels que $BF, MTT$ ou $BV$ ainsi que la fonction des résidus $R(t)$ sont actuellement estimés comme suit, dans le cas de l'imagerie de perfusion par résonance magnétique nucléaire.

Pour chaque voxel, le signal de perfusion expérimental $S_{exp}(t)$ échantillonné aux instants de mesure $t_i$, $i = 1, N$, est converti en une courbe de concentration $C(t)$. A partir de la courbe de concentration $C(t)$, et en supposant connue la fonction d'entrée artérielle théorique associée $C_a(t)$, le produit $BF \cdot R(t)$ est estimé par déconvolution numérique.

Diverses approches ont été envisagées pour obtenir les fonctions d'entrée artérielles théoriques $C_a(t)$ afin de déconvoluer ensuite les courbes de concentration $C(t)$.

Dans une première approche, une fonction d'entrée artérielle globale expérimentale est choisie manuellement par le praticien. Elle peut être mesurée, par exemple, au niveau d'une artère proche de l'organe à étudier.

Selon une autre approche, des fonctions d'entrée artérielle locales sont obtenues automatiquement à partir d'images de perfusion, à l'aide de techniques de traitement du signal et de critères de sélection. On recherche par exemple la « meilleure » fonction dans le voisinage immédiat du voxel tissulaire courant où l'on souhaite estimer les paramètres hémodynamiques ou les fonctions de répartition complémentaires. L'objet de cette approche est d'obtenir au final des estimations moins biaisées et plus précises en s'affranchissant, du moins en partie, des problèmes de délai et de dispersion.

Une fois les fonctions d'entrée artérielle $C_a(t)$ obtenues, il est nécessaire de déconvoluer les courbes de concentration afin d'estimer la fonction des résidus R(t) et d'en déduire les paramètres hémodynamiques, tels que le flux sanguin $BF,$ le temps moyen de transit $MTT$ ou le volume sanguin $BV.$

Pour effectuer l'opération de déconvolution de la courbe de concentration expérimentale $C(t)$ par la fonction d'entrée artérielle $C_a(t)$ théorique donnée par les méthodes décrites précédemment, deux approches sont généralement utilisées.

Une première approche est une déconvolution paramétrique.

On cherche à modéliser le voxel par plusieurs compartiments distincts échangeant entre eux l'agent de contraste. De cette manière, des modèles paramétriques $R(t, \Theta_R)$ pour des fonctions des résidus sont introduits, $\Theta_R$ étant le vecteur des paramètres dudit modèle, lesdits modèles dépendant des paramètres hémodynamiques du voxel. Ces modèles sont ajustés aux signaux expérimentaux, par exemple par la méthode de Bayes.

Plusieurs modèles paramétriques connus permettent alors de décrire les courbes de concentration de l'agent de contraste dans chaque voxel. A titre d'exemple non limitatif, le modèle le plus simple est le modèle de Kety, utilisant le postulat suivant : chaque voxel est séparé en deux compartiments, l'espace intravasculaire et l'espace extravasculaire, lesdits compartiments occupant chacun respectivement une fraction de voxel $v_a$ et $v_e$, également et respectivement caractérisés par une concentration en agent de contraste $C_a(t)$ et $C_e(t)$. Ainsi, le modèle de Kety s'écrit généralement sous la forme intégrale : $C(t)=K_{trans}C_a(t)\otimes e^{-k_{ep}t}$, où $K_{trans}$ correspond au flux sanguin, $k_{ep}$ est la constante de temps caractérisant le transfert entre les deux compartiments, $\otimes$ désigne le produit de convolution et $C_a(t)$ est la fonction d'entrée artérielle.

L'ensemble des paramètres hémodynamiques d'un tel modèle est $\theta_{hem}=\{K_{trans},k_{ep}\}$ . Par ailleurs, on définit le

$$v_e = \frac{K_{trans}}{k_{ep}}$$

volume sanguin $v_e$ , autre paramètre hémodynamique, par le théorème du volume central comme suivant                .

D'autres modèles permettent de rendre compte plus finement des processus physiologiques à l'œuvre dans le voxel, en introduisant des fonctions des résidus plus complexes, ainsi que des paramètres hémodynamiques supplémentaires, tels que le modèle de Kety-Tofts ou de Tofts-Kermode étendu, le modèle de St Lawrence et Lee, etc.

Une fois le modèle choisi et par voie de conséquence le type de fonction des résidus fixé, la fonction d'entrée artérielle doit également être choisie. Le choix de ladite fonction se révèle déterminant pour l'estimation des paramètres hémodynamiques : généralement, le praticien ou le système d'imagerie choisit la courbe de concentration de l'agent de contraste d'un voxel situé au niveau d'une artère comme fonction d'entrée artérielle de tous les voxels du volume étudié. Par la suite, le produit de convolution entre la fonction d'entrée artérielle et la fonction des résidus est calculé numériquement en le discrétisant sur la grille temporelle d'acquisition des données de perfusions. La concentration théorique de l'agent de contraste $C(t)$ à chaque instant de l'acquisition $t_i$ s'écrit en fonction des paramètres hémodynamiques $\theta_{hem}$ : $C(t_i) = f(t_i, \theta_{hem})$ . Différentes méthodes sont applicables pour estimer les paramètres hémodynamiques $\theta_{hem}$ à partir des courbes de concentration en agent de contraste au sein de chaque voxel. Un procédé d'estimation d'un tel paramètre mis en œuvre par une unité de traitement d'un système, tel que celui décrit en liaison avec les figures 1 ou 2, comporte une étape pour minimiser la somme quadratique des différences entre le modèle et les mesures

$$\hat{\theta}_{hem} = \arg\min \sum_i \left[ C^{exp}(t_i) - f(t_i, \theta_{hem}) \right]^2$$

expérimentales selon la formule suivante :                . La minimisation est réalisée numériquement, avantageusement à l'aide de l'exécution d'un algorithme d'optimisation non linéaire.

Après avoir estimé des paramètres hémodynamiques de chaque voxel d'intérêt, des résultats peuvent être présentés sous la forme de cartes de paramètres, mettant alors en évidence différents types de tissus, à savoir sains ou pathologiques, selon les valeurs desdits paramètres.

Une deuxième approche est une approche plus générale basée sur la déconvolution non paramétrique : aucune hypothèse de modèle n'est faite pour la fonction des résidus.

Le modèle standard de convolution $C(t)=BFC_a(t)\otimes R(t)$ est tout d'abord discrétisé temporellement selon le modèle suivant : $c = BFAr$ où,

- c est le vecteur contenant la concentration de l'agent de contraste aux instants d'acquisition,
- *BF* est le flux sanguin (« Blood Flow » selon une terminologie anglo-saxonne),
- *r* est le vecteur de la fonction des résidus discrétisée,
- A est la matrice de convolution dépendant de la fonction d'entrée artérielle.

Comme explicité précédemment dans le cas de la première approche décrite, la fonction d'entrée artérielle doit être choisie correctement par rapport aux voxels étudiés, pour permettre la construction de la matrice de convolution A.

Une telle discrétisation peut se faire, par exemple, suivant l'approximation de la méthode des rectangles :

$$\forall i = 1, N, \quad C(t_i) = BF \cdot \int_0^{t_i} C_a(\tau) \cdot R(t-\tau)d\tau \approx BF \cdot \Delta t \cdot \sum_{k=0}^{i} C_a(t_i) \cdot R(t_i - t_k)$$

où $\Delta t$ est la période d'échantillonnage. On se ramène alors à un système linéaire $Ad=c$ en posant

$$A = \Delta t. \begin{pmatrix} C_a(t_1) & 0 & \dots & 0 \\ C_a(t_2) & C_a(t_1) & \ddots & \vdots \\ \vdots & \vdots & \ddots & 0 \\ C_a(t_N) & C_a(t_{N-1}) & \dots & C_a(t_1) \end{pmatrix} \quad b = \begin{vmatrix} R(t_1) \\ R(t_2) \\ \dots \\ R(t_N) \end{vmatrix} \quad c = \begin{vmatrix} C(t_1) \\ C(t_2) \\ \dots \\ C(t_N) \end{vmatrix}$$

$$d = BF.b$$

En pratique, la matrice A est très mal conditionnée et presque singulière, de sorte qu'on ne peut pas inverser numériquement ce système linéaire sous peine d'obtenir des solutions dénuées de sens et des estimations aberrantes. Il faut donc recourir à diverses méthodes pour obtenir par exemple une pseudo-inverse $\tilde{A}^{-1}$ de la matrice $A$ et par la suite une estimation $\hat{d}$ de $d$ par $\hat{d} = \tilde{A}^{-1}.c$. Parmi ces méthodes d'obtention d'une pseudo-inverse mises en œuvre par l'unité de traitement d'un système d'imagerie tel que celui décrit en liaison avec les figures 1 ou 2, on peut citer la décomposition en valeurs singulières tronquée (Truncated Singular Value Décomposition, - (T)SVD - ou Simple Singular Value Décomposition, -sSVD - selon des terminologies anglo-saxonnes) et la déconvolution de Hunt dans le domaine fréquentiel. Le document ne saurait être limité aux seules techniques citées.

De manière plus générale, un tel procédé pour estimer une quantité d'intérêt consiste à minimiser un critère du type $||Ad-c||^2 + ||\Gamma d||^2$ où $||\Gamma d||^2$ est un terme de régularisation privilégiant certaines solutions et obtenir une estimation de $d$ par

$$\hat{d} = \arg\min_{d}\left( \left\|Ad - c\right\|^2 + \left\|\Gamma d\right\|^2 \right)$$

. Parmi ces méthodes, nous pouvons citer la régularisation de Tikhonov, l'utilisation de la transformée en ondelettes, etc.

Une fois $\hat{d}$ obtenue, l'unité de traitement peut mettre en œuvre un procédé pour estimer $BF$ de $BF$ par $\widehat{BF} = \hat{d}(t_1) = \hat{d}(0)$ puisque, par définition, $R(0)=1$. On trouve de nombreuses variantes de ces méthodes à base de fonction(s) d'entrée artérielle : par exemple, les fonctions d'entrée artérielle expérimentales peuvent être préalablement ajustées à un modèle théorique paramétrique ou semi-paramétrique $C_a(t,\Theta_a)$ où $\Theta_a$ est un vecteur de paramètres, afin d'accroître artificiellement le rapport signal sur bruit.

D'autres paramètres hémodynamiques peuvent être estimés une fois la fonction des résidus R(t) elle-même, tels que le temps de transit moyen (MTT ou encore connu sous la dénomination anglo-saxonne « Mean Transit Time ») à travers le voxel, pouvant s'exprimer grâce à la formule suivante : $MTT = \int R(t)dt \approx \Delta t \sum r_i$. Le volume sanguin contenu dans le voxel (BV ou encore connu sous la dénomination anglo-saxonne « Blood Volume ») peut également être calculé au moyen du théorème du volume central : $BV = BF.MTT$.

Aujourd'hui, les contraintes techniques des appareils d'imagerie par Résonance Magnétique Nucléaire , tels qu'illustrés à titre d'exemple non limitatif par les figures 1 et 2, ne permettent d'obtenir et/ou acquérir des images à champ d'acquisition limité à quelques dizaines de centimètres carrés. Le volume délivré par les différentes acquisitions et analyses de séquences d'images est ainsi restreint et les techniques connues d'analyse d'imagerie de perfusion, notamment par Résonance Magnétique Nucléair, sont adaptées pour l'étude d'un « petit » organe, tel qu'à titre d'exemples non limitatifs, le cerveau ou le cœur, ou l'étude d'une partie restreinte du corps. En revanche, de telles techniques deviennent inadéquates ou inopérantes pour l'étude d'organes de tailles plus importantes ne pouvant être couverts par les champs d'acquisition d'un appareil d'imagerie, tels qu'à titre d'exemple non limitatif la moelle épinière, ou encore l'étude du corps entier, notamment à titre d'exemple non limitatif, pour suivre l'apparition et/ou l'évolution de métastases pouvant être localisées dans tout le corps.

Une manière « naïve » de résoudre ce problème serait de réaliser autant d'examens de perfusion, notamment d'acquisitions, qu'il est nécessaire pour couvrir en totalité le volume qu'on désire analyser. Dans ce cas, la durée totale d'acquisition, notamment celle passée par le patient dans des appareils d'imagerie, serait multipliée par le nombre de sous-volumes nécessaires pour analyser le volume d'intérêt en entier, augmentant considérablement la durée totale de l'examen : de tels examens deviendraient très lourds et seraient plus difficiles à supporter par le patient. En principe, les analyses par tomodensitométrie peuvent consister à mesurer l'absorption des rayons X par les tissus des différents voxels. En appliquant le protocole décrit précédemment, la dose de rayonnements reçue par un patient, lors de l'exploitation d'un tel protocole, serait considérablement augmentée, rendant l'examen très dangereux pour ledit patient du fait d'une surexposition auxdits rayonnements. La demande de brevet US 2011/0110488 A1 divulgue un exemple d'appareil par Tomodensimétrie pour l'acquisition de données de perfusion et mentionne la possibilité d'utiliser une table mobile et de changer la position du patient dans le scanner entre différents battements de cœur dans le contexte d'acquisition de données de perfusion pour la reconstruction d'une fonction d'entrée artérielle et l'estimation d'une quantité d'intérêt d'un système dynamique artère/tissu/veine d'un volume élémentaire du cœur. Il n'y a cependant aucun détail quant au traitement des données de perfusion lié à ce mode d'acquisition.

En outre, pour chaque acquisition de sous-volume, une injection d'agent de contraste serait nécessaire. Bien qu'un tel agent de contraste permette d'améliorer la qualité des diagnostics par imagerie médicale par Résonance Magnétique, la toxicité à forte concentration d'un tel agent de contraste peut se révéler problématique pour un patient. La quantité d'agent de contraste qu'un patient peut supporter étant limitée, une telle approche est inadaptée, voire potentiellement dangereuse.

D'autres protocoles expérimentaux, tels que, à titre d'exemple non limitatif le protocole expérimental d'acquisition des professeurs Rahmouni et Luciani, ont été mis en place pour permettre l'étude de l'imagerie de perfusion du corps entier, plus particulièrement en imagerie par Résonance Magnétique (voir par exemple les articles de Luciani A. et al., "Hématologie : applications IRM corps entier", Journal de Radiologie, 90(10), p. 1338 (2009) et de Lin S. et al., "IRM corps entier en haute resolution avec injection dynamique de gadolinium dans les syndromes lymphoproliferatifs", Journal de Radiologie, 88(10), p. 1445(2007)) . De tels protocoles ont été développés notamment pour l'étude des myélomes, une forme de cancer touchant les cellules de la moelle épinière. La taille de la colonne vertébrale, et par voie de conséquence de la moelle épinière, dépassant largement le champ de vision et/ou d'acquisition des appareils d'imagerie par Résonance Magnétique, les méthodes classiques utilisées en principe pour l'imagerie de perfusion de cet organe ne sont pas adaptées.

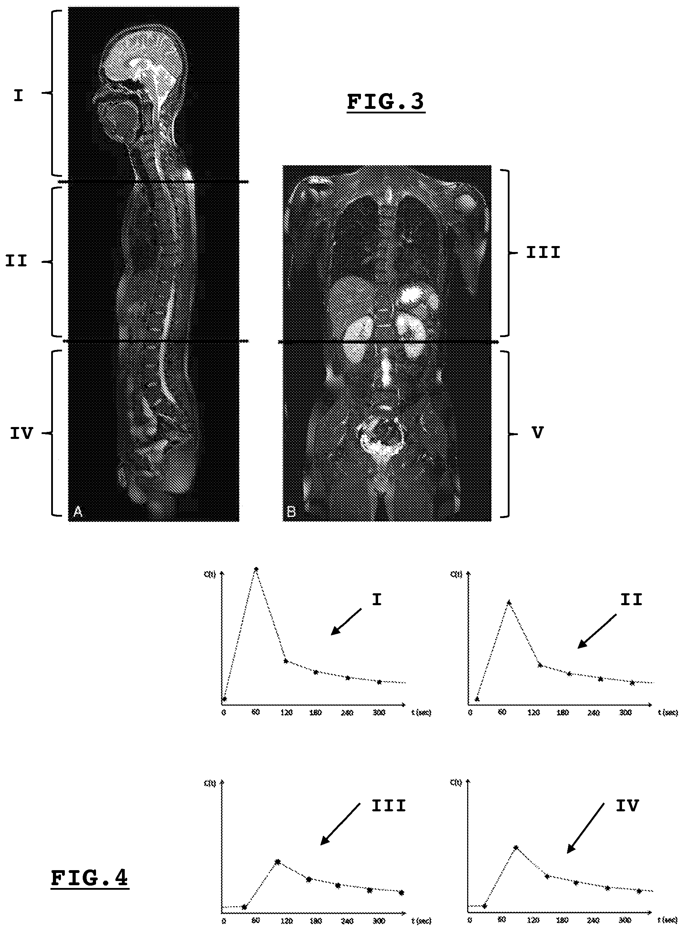

Pour obtenir un suivi dynamique de l'agent de contraste dans l'intégralité de la colonne vertébrale consécutivement à une injection unique dudit agent, l'acquisition peut se faire, par exemple, selon le protocole suivant : un patient est en principe allongé sur une table mobile, généralement motorisée, se déplaçant dans le champ de vision de l'appareil d'imagerie par Résonance Magnétique. Après qu'un premier volume, également connu sous la terminologie « Station », correspondant à un champ d'acquisition de l'appareil d'imagerie, a été scanné par l'appareil d'imagerie par Résonance Magnétique, le patient est déplacé au sein dudit appareil d'imagerie au moyen de ladite table mobile, de manière à ce qu'un deuxième volume ou station contenu dans le champ d'acquisition, éventuellement adjacent au premier volume ou station précédemment analysé, fasse l'objet d'une acquisition. A titre d'exemple non limitatif, un tel protocole est illustré en liaison avec la figure 3. Selon cet exemple, cinq stations I à V, trois stations sagittales I, II, IV et deux stations coronales III, V successives font l'objet d'acquisitions, lesdites stations étant numérotées dans l'ordre d'acquisitions: une période de soixante secondes est nécessaire pour échantillonner les cinq volumes et revenir au premier volume. Une telle période se décompose en une sous-période d'acquisition des images d'environ trente neuf secondes et une sous-période d'environ vingt et une secondes de mouvement de la table mobile. Chaque voxel d'intérêt du volume global est échantillonné toutes les soixante secondes. Les courbes de concentration résultantes ont ainsi une résolution temporelle de soixante secondes.

Selon un tel protocole, les données de perfusion acquises et associées à chaque volume ou station sont par la suite traitées afin d'estimer une quantité d'intérêt, telle qu'à titre d'exemples non limitatifs, des paramètres hémodynamiques. Une approche « naturelle » consiste à considérer chacune des stations indépendamment les unes des autres, en leur appliquant des méthodes classiques telles que décrites précédemment, à savoir des approches paramétriques ou non paramétriques. Pour l'approche paramétrique par exemple, un modèle, tel qu'à titre d'exemple non limitatif le modèle de Ketty, et une fonction d'entrée artérielle peuvent être choisis pour chaque station. Les paramètres hémodynamiques, tels que par exemple les paramètres $K_{Trans}$, $k_{ep}$ et $v_e$, sont ainsi estimés et les cartes des paramètres associées peuvent être également produites.

Toutefois, cette méthode « naturelle » donne lieu à des résultats peu ou non comparables d'une station à une autre. Par exemple, la valeur moyenne du $K_{Trans}$ mesurée dans la vertèbre lombaire L1 peut varier du simple au double selon la station à partir de laquelle l'acquisition est faite. Cette variabilité peut être observée également pour les autres paramètres $k_{ep}$ et $v_e$. Les résultats ainsi obtenus par l'utilisation d'un tel protocole en appliquant des procédés d'estimation connus, ne sont par conséquent pas reproductibles, rendant les données obtenues selon ce protocole d'acquisition peu exploitables, voire encore totalement inexploitables. En effet, en appliquant une analyse indépendante pour chaque station, on occulte le fait que les voxels de chaque volume ont été acquis successivement, à des instants différents. Par exemple, pour le protocole décrit précédemment, la station IV est acquise quinze secondes après la station III. La fonction d'entrée artérielle propre à chaque station n'est donc pas échantillonnée aux mêmes instants, et chaque station semble avoir une fonction d'entrée artérielle différente. En conséquence, la fonction d'entrée artérielle de chaque station est modélisée selon un modèle différent, valable uniquement aux instants d'acquisition de la station à laquelle il est attaché, comme décrit en liaison avec la figure 4. La figure 4 illustre des exemples de fonctions d'entrée artérielle produites et/ou choisies pour chaque station I à IV, lesdites fonctions étant représentées sous la forme d'une courbe de concentration d'un agent de contraste en fonction du temps au niveau d'une artère choisie. Pour chaque station I à IV, une fonction d'entrée artérielle indépendante est déterminée automatiquement et/ou manuellement. En effet, la courbe de concentration d'une fonction d'entrée artérielle est caractérisée par une montée rapide suivie d'une décroissance tout aussi rapide, avec un temps caractéristique de quelques dizaines de secondes seulement. Ensuite, une phase de décroissance plus lente est observée avec un temps caractéristique plus long. La résolution temporelle des courbes de concentration étant de l'ordre de la minute, il n'est pas possible d'échantillonner correctement les variations rapides. Les différentes fonctions d'entrée artérielle déterminées dans chaque station ne reproduisent donc pas toute la dynamique présente en réalité et les dernières stations acquises sont davantage affectées par cet effet que les premières. Ainsi, la non simultanéité des acquisitions entre chaque station et la faible résolution temporelle sont autant de raisons conduisant à des estimations de paramètres hémodynamiques non reproductibles d'une station à l'autre, rendant l'analyse d'organes de taille importante, tels que la moelle épinière, par les techniques d'imagerie de perfusion connues, inadaptées et inopérantes.

L'invention permet de répondre à tout ou partie des inconvénients soulevés par les solutions connues.

Parmi les nombreux avantages apportés par l'invention, nous pouvons mentionner que celle-ci permet de :

- s'affranchir de l'échantillonnage temporel de la fonction d'entrée artérielle, en choisissant une fonction d'entrée artérielle commune pour toutes les stations analysées ;
- préserver la santé d'un patient en injectant une dose unique d'agent de contraste pouvant permettre l'analyse d'organes de taille importante présents sur une pluralité de stations ou du corps entier ;
- effectuer des analyses par imagerie de perfusion, d'organes de taille importante, tels qu'à titre d'exemples non limitatifs la moelle épinière, la taille desdits organes étant supérieure au champ d'acquisition standard des appareils d'analyse d'imagerie par Résonance Magnétique ;
- offrir la possibilité de vérifier des fonctions d'entrée artérielle construites ;
- proposer une solution indépendante de toutes les techniques d'estimation de paramètres hémodynamiques ;
- permettre l'affichage et une vision globale de cartes d'organes présents sur plusieurs stations ;
- donner des résultats reproductibles, comparables d'une station à une autre.

A cette fin, il est notamment prévu un procédé, selon la revendication indépendante 1, pour produire une estimation d'une quantité d'intérêt d'un système dynamique artère/tissu/veine d'un volume élémentaire - dit voxel - d'un organe d'un patient. Un tel procédé est destiné à être mis en œuvre par des moyens de traitement d'une unité de traitement d'un système d'analyse d'imagerie de 2. perfusion par résonance magnétique, et comporte une étape pour estimer ladite quantité d'intérêt pour une station parmi une pluralité de stations à partir de données de perfusion liées à ladite station. Selon l'invention, un tel procédé comporte une étape pour construire une fonction d'entrée artérielle commune à partir de données de perfusion liées à au moins une station parmi ladite pluralité de stations, chaque station étant un volume correspondant à un champ d'acquisition défini par un appareil d'imagerie médicale par résonance magnétique

. En outre, ladite étape pour estimer la quantité d'intérêt pour une station de ladite pluralité exploite , pour toute station de ladite pluralité de stations, Ladite fonction d'entrée artérielle commune construite et les données de perfusion liées à ladite station pour estimer la quantité d'intérêt , ladite station comprenant le volume élémentaire de l'organe du patient

En variante, l'étape 120 pour construire une fonction d'entrée artérielle commune peut être mise en œuvre à partir des données de perfusion liées à au moins deux stations parmi ladite pluralité de stations.

Selon un mode de réalisation préféré, l'invention prévoit que l'étape pour construire une fonction d'entrée artérielle commune puisse comporter une étape pour déterminer une fonction d'entrée artérielle propre à chaque station parmi ladite au moins une station, la fonction d'entrée artérielle commune étant construite à partir de ladite fonction d'entrée artérielle propre.

Avantageusement, lorsque le système d'analyse d'imagerie de perfusion par résonance magnétique comporte des moyens de restitution à un utilisateur dudit système, lesdits moyens de restitution coopérant avec l'unité de traitement, ledit procédé peut comporter une étape subséquente pour déclencher une restitution de la fonction d'entrée artérielle commune par lesdits moyens de restitution.

Pour améliorer la qualité des données de perfusion, l'invention prévoit que le procédé puisse comporter une étape préalable de prétraitement des données de perfusion, ladite étape étant agencée pour corriger lesdites données de perfusion.

Selon un deuxième objet, l'invention concerne un procédé, selon la revendication 6, pour produire une estimation d'une quantité d'intérêt d'un système dynamique artère/tissu/veine d'une région d'intérêt, ladite région comportant au moins un voxel. Selon l'invention, ladite quantité d'intérêt est estimée par voxel au moyen d'un procédé conforme au premier objet de l'invention.

Pour permettre l'analyse d'un organe dont la taille ou la configuration excède le champ d'acquisition de l'appareil d'analyse par imagerie de perfusion, ladite région d'intérêt peut s'étendre sur plusieurs stations parmi ladite pluralité de stations.

Avantageusement, lorsque le système d'analyse d'imagerie de perfusion par résonance magnétique comporte des moyens de restitution à un utilisateur dudit système, lesdits moyens de restitution coopérant avec l'unité de traitement, ledit procédé peut comporter une étape subséquente pour déclencher une restitution de ladite quantité d'intérêt estimée pour les voxels de la région d'intérêt par lesdits moyens de restitution.

Selon un troisième objet, l'invention concerne une unité de traitement, selon la revendication indépendante 9, comportant des moyens pour communiquer avec le monde extérieur et des moyens de traitement, coopérant avec des moyens de mémorisation. Les moyens pour communiquer sont adaptés à recevoir du monde extérieur des données de perfusion liées avec une pluralité de stations, chaque station étant un volume correspondant à un champ d'acquisition défini par un appareil d'imagerie médicale, et les moyens de mémorisation comportent des instructions exécutables ou interprétables par les moyens de traitement dont l'interprétation ou l'exécution desdites instructions par lesdits moyens de traitement provoque la mise en œuvre d'un procédé conforme à l'invention.

Pour permettre à un praticien d'effectuer un diagnostic et prendre une décision rapide, les moyens pour communiquer de l'unité de traitement conforme à l'invention peuvent délivrer une quantité d'intérêt estimée selon un format approprié à des moyens de restitution aptes à la restituer à un utilisateur.

Selon un quatrième objet, l'invention concerne un système d'analyse d'imagerie de perfusion, selon la revendication 11, comportant une unité de traitement conforme à l'invention et des moyens de restitution adaptés à restituer à un utilisateur une quantité d'intérêt estimée selon un procédé conforme à l'invention et mis en œuvre par ladite unité de traitement.

Selon un cinquième objet, l'invention concerne un produit programme d'ordinateur, selon la revendication 12, comportant une ou plusieurs instructions interprétables ou exécutables par les moyens de traitement d'une unité de traitement conforme à l'invention. Ladite unité de traitement comporte en outre des moyens de mémorisation ou coopérant avec de tels moyens de mémorisation, ledit programme étant chargeable dans lesdits moyens de mémorisation. L'interprétation ou l'exécution desdites instructions par lesdits moyens de traitement provoque la mise en œuvre d'un procédé pour estimer une quantité d'intérêt conforme à l'invention.

D'autres caractéristiques et avantages apparaîtront plus clairement à la lecture de la description qui suit et à l'examen des figures qui l'accompagnent parmi lesquelles :

- les figures 1 et 2, précédemment décrites, présentent deux variantes de réalisation d'un système d'analyse d'imagerie médicale ;
- La figure 3, précédemment décrite, présente des images de perfusion, obtenues par un appareil d'imagerie par Résonance Magnétique Nucléaire, en lien avec plusieurs stations imagées d'un être humain après injection d'un agent de contraste;
- la figure 4, précédemment décrite, présente des exemples de fonctions d'entrée artérielle déterminées pour quatre stations, sous la forme de courbes de concentration $C(t)$ d'un agent de contraste circulant au sein d'un voxel d'une artère en fonction du temps ;

- les figures 5a et 5b présentent des organigrammes simplifiés de procédés conformes à l'invention ;
- la figure 6 présente un exemple d'une fonction d'entrée artérielle construite à partir des données de perfusion de quatre stations, conformément à l'invention ;
- la figure 7 présente cinq cartes relatives au flux sanguin estimé à partir d'une pluralité de stations conformément à l'invention ;
- la figure 8 présente une carte consolidée relative audit flux sanguin estimé.

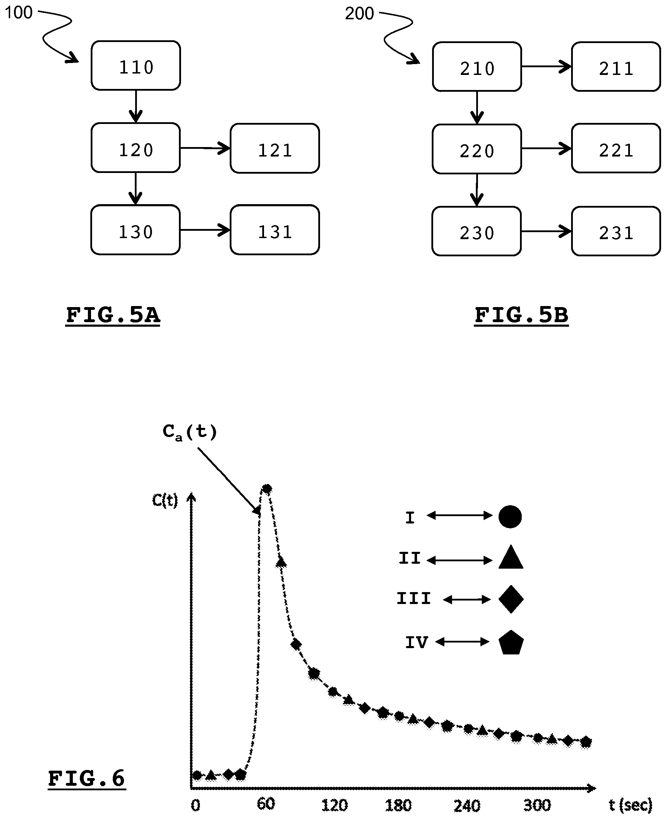

La figure 5a décrit un exemple d'algorithme simplifié d'un procédé 100 - conforme à l'invention - pour produire une estimation d'une quantité d'intérêt d'un système dynamique artère/tissu/veine d'un volume élémentaire que l'on nomme voxel - d'un organe. Un tel procédé 100 peut être mis en œuvre par une unité de traitement d'un système d'analyse d'imagerie de perfusion, tel que le système décrit en liaison avec les figures 1 ou 2 et adapté en conséquence.

Le procédé 100 conforme à l'invention comporte principalement une étape 130 pour estimer une quantité d'intérêt, à titre d'exemples non limitatifs un paramètre hémodynamique ou la fonction des résidus, à partir de données de perfusion en lien avec une pluralité de stations. On entend au sens de l'invention par « station », un volume correspondant au champ d'acquisition d'un appareil d'imagerie médicale, tel que l'appareil 1 du système d'analyse d'imagerie de perfusion décrit en liaison avec les figures 1 ou 2. Ainsi, chaque station est liée ou associée à un volume correspondant à un champ d'acquisition défini par un appareil d'imagerie médical. Ledit champ d'acquisition est associé à une position particulière de la table mobile de l'appareil d'imagerie médicale, sur laquelle le patient est en principe allongé. On entend au sens de l'invention par « données de perfusion », l'ensemble des images ou signaux acquis à plusieurs instants par un appareil d'imagerie médicale par résonance magnétique dans le but d'étudier l'évolution d'un agent de contraste préalablement injecté dans l'organisme d'un patient. De telles données peuvent avantageusement consister en des séquences d'images, telles que les séquences d'images 12 décrites en liaison avec les figures 1 ou 2. Comme précisé précédemment en liaison avec les figures 1 et 2, les données de perfusion peuvent avantageusement être stockées dans un serveur lors de leur acquisition, pour être traitées plus tard par un utilisateur d'un système d'analyse d'imagerie, avantageusement praticien. Les données de perfusion sont avantageusement liées ou associées à une station particulière.

Selon l'invention, préalablement à l'estimation de paramètres hémodynamiques en 130, pour s'affranchir de l'échantillonnage temporel de la fonction d'entrée artérielle, un tel procédé 100 comporte une étape 120 pour construire une fonction d'entrée artérielle commune à partir de données de perfusion en lien avec au moins une station parmi ladite pluralité de stations. Toutefois, la construction d'une telle fonction d'entrée artérielle selon l'étape 120 ne saurait être limitée aux données de perfusion en lien avec une seule station : en effet, afin, par exemple, d'ajuster un modèle de fonction d'entrée artérielle au plus proche de la réalité et affiner la construction de ladite fonction d'entrée artérielle commune, les données de perfusion peuvent être liées ou associées à deux stations ou plus, à savoir autant que de stations disponibles. A titre d'exemple non limitatif, comme décrit en liaison avec la figure 3, les données de perfusion peuvent provenir des cinq stations I à V pour construire une fonction d'entrée artérielle commune.

La figure 6 décrit un exemple d'une fonction d'entrée artérielle construite à partir de quatre stations I à IV parmi les cinq acquises, lesdites stations étant définies par la figure 3 et acquises selon le protocole expérimental d'acquisition des professeurs Rahmouni et Luciani.

De façon générale, la fonction d'entrée artérielle commune construite est avantageusement unique pour toutes les stations : une telle fonction d'entrée artérielle peut être décrite par une fonction analytique donnée sous la forme $C_a(t, \theta_a)$ où $\theta_a$ est l'ensemble des paramètres de la fonction d'entrée artérielle. De tels paramètres $\theta_a$ sont estimés en 120 à partir des données de perfusion des différentes stations et sont les mêmes pour tous les voxels de toutes les stations. Une telle estimation des paramètres $\theta_a$ peut être réalisée, à titre d'exemple non limitatif, en ajustant le modèle défini $C_a(t, \theta_a)$ sur des courbes de concentrations de l'agent de contraste issues d'artères choisies dans toutes les stations. Lesdites artères peuvent être choisies manuellement ou automatiquement selon le protocole choisi. En variante ou en complément, les paramètres $\theta_a$ peuvent également être ajustés conjointement à des paramètres hémodynamiques $\theta_{hem}$ des voxels. Selon un mode de réalisation préféré, l'invention prévoit que l'étape 120 pour construire une fonction d'entrée artérielle commune puisse comporter une étape pour déterminer, manuellement ou automatiquement, une fonction d'entrée artérielle propre ou dédiée à une station parmi ladite au moins une station, la fonction d'entrée artérielle commune étant construite à partir de ladite fonction d'entrée artérielle propre. Comme précisé précédemment, afin d'ajuster un modèle de fonction d'entrée artérielle au plus proche de la réalité et affiner la construction de ladite fonction en estimant les paramètres $\theta_a$, les données de perfusion peuvent être liées ou associées à deux stations ou plus, à savoir jusqu'à autant de stations que disponibles. Un tel mode de réalisation préféré est décrit en liaison avec la figure 6. Dans cet exemple, l'étape 120 pour construire une fonction d'entrée artérielle commune $C_a(t)$ consiste à ajuster ladite fonction aux valeurs mesurées de concentrations d'agent de contraste aux instants d'échantillonnage au sein de chaque station, parmi les stations I à IV. Comme précisé précédemment, ladite fonction d'entrée artérielle commune peut être décrite par une fonction analytique donnée sous la forme $C_a(t, \theta_a)$ où $\theta_a$ est l'ensemble des paramètres de la fonction d'entrée artérielle. A titre d'exemple non limitatif mais préféré, une telle fonction analytique peut correspondre à une

$$C_a\left(t, \Theta_a\right) \begin{cases} 0 \ si \ t < \tau \\ A_1 e^{-k1(t-\tau)} + A_2 e^{-k2(t-\tau)} \ si \ t < \tau \end{cases}$$

fonction bi-exponentielle telle que : où $\theta_a = \{A_1, A_2, k_1, k_2, \tau\}$. Un tel modèle est particulièrement adapté pour la mise en œuvre d'un procédé conforme à l'invention, puisqu'il reproduit correctement les caractéristiques temporelles de la courbe de fonction d'entrée artérielle $C_a(t)$, c'est-à-dire une montée brusque, puis une décroissance rapide et enfin une décroissance plus lente.

En variante ou en complément, l'invention prévoit également que l'étape 120 pour construire une fonction d'entrée artérielle commune consiste à choisir, dans une base de données de fonctions d'entrée artérielle existantes, lesdites fonctions étant préalablement mesurées avec une résolution temporelle suffisante dans une population de patients de référence, une fonction d'entrée artérielle commune. Le choix d'une telle fonction d'entrée artérielle suréchantillonnée pourra être réalisé en utilisant les données de perfusion de chaque station.

Avantageusement, lorsque le système d'analyse d'imagerie de perfusion décrit en liaison avec les figures 1 ou 2, comporte des moyens de restitution 5, tels qu'à titre d'exemple non limitatif une interface homme-machine, tel qu'un écran ou tout autre moyen équivalent, à un utilisateur 6 dudit système, lesdits moyens de restitution 5 coopérant avec l'unité de traitement 4, ledit procédé peut comporter une étape 121 subséquente pour déclencher une restitution de la fonction d'entrée artérielle commune ainsi construite par lesdits moyens de restitution. En variante ou en complément, une telle étape 121 peut par ailleurs déclencher une restitution des voxels des données de perfusion utilisés pour construire la fonction d'entrée artérielle commune. Une telle étape 121 permet notamment, lorsque les voxels ont été sélectionnés automatiquement pour construire la fonction d'entrée artérielle commune, de valider la pertinence desdits voxels sélectionnés, à savoir que les voxels sélectionnés correspondent effectivement à des voxels d'intérêt au niveau des artères.

En outre, l'étape 130 pour estimer la quantité d'intérêt exploite la fonction d'entrée artérielle commune construite : une telle fonction d'entrée artérielle est valable pour toutes les stations à tous les instants. L'étape 130 pour estimer la quantité d'intérêt peut consister en la mise en œuvre par l'unité de traitement de toutes techniques connues d'estimation de paramètres hémodynamiques, avantageusement des méthodes paramétriques ou non-paramétriques, telles que décrites précédemment.

Selon l'approche paramétrique, pour un modèle de microcirculation donné, la fonction des résidus peut être décrite par une fonction analytique donnée sous la forme $R(t, \theta_{hem})$ où $\theta_{hem}$ est l'ensemble des paramètres hémodynamiques. A titre d'exemples non limitatifs, une méthode d'optimisation des paramètres de la fonction pourra avantageusement être exécutée, telle que la minimisation des moindres carrés, avec ou sans contrainte, ou encore une méthode d'estimation bayésienne.

En variante ou en complément, selon l'approche non paramétrique, une matrice de convolution peut être construite à partir de la fonction d'entrée artérielle construite commune en utilisant une grille temporelle plus fine que celle du protocole d'acquisition, de manière à échantillonner correctement les différentes échelles de temps présentes dans ladite fonction d'entrée artérielle. A titre d'exemples non limitatifs, la méthode SVD (Singular Value Décomposition selon une terminologie anglo-saxonne) pourra être exécutée. Toutefois, la méthode de déconvolution bayésienne sera préférée eu égard à la précision particulièrement avérée des estimations produites.

En complément, lorsque le système d'analyse d'imagerie de perfusion décrit en liaison avec les figures 1 ou 2, comporte des moyens de restitution 5, tels qu'à titre d'exemple non limitatif une interface homme-machine ou tout autre moyen équivalent, à un utilisateur 6 dudit système, lesdits moyens de restitution coopérant avec l'unité de traitement 4, ledit procédé peut comporter une étape 131 subséquente pour déclencher une restitution de la quantité d'intérêt estimée, telle que par exemple les paramètres hémodynamiques 14, selon un format approprié. A titre d'exemples non limitatifs, un tel format peut être une valeur, une couleur au sein d'une palette déterminée pour exprimer l'intensité de ladite quantité d'intérêt estimée, ou tout autre moyen équivalent.

Pour accroître la pertinence des données de perfusion, l'invention prévoit que le procédé puisse comporter une étape 110 préalable de prétraitement des données de perfusion, ladite étape consistant notamment à corriger des artefacts ou à appliquer tout autre filtre correctif.

L'imagerie par Résonance Magnétique, comme toutes les autres techniques d'imagerie médicale, n'échappe pas à la constitution de fausses images : les artéfacts. Les artéfacts sont des images observables ne représentant aucune réalité anatomique. Bien souvent, on cherche à les éviter ou à les minimiser en modifiant certains paramètres d'acquisition ou de reconstructions. De tels artefacts peuvent être de différentes natures. En principe, à titre d'exemples non limitatifs, trois corrections sont généralement appliquées pour améliorer la qualité des données de perfusion : une correction des mouvements du patient ou bien des mouvements dus à la respiration, les battements cardiaques et les flux sanguins, une correction du champ de vision de l'appareil d'imagerie de perfusion et/ou un débruitage des images.

La figure 5b décrit un exemple d'algorithme simplifié d'un procédé 200 - conforme à l'invention - pour produire une estimation d'une quantité d'intérêt d'un système dynamique artère/tissu/veine d'une région d'intérêt.

Un procédé 200 est agencé pour produire une estimation d'une quantité d'intérêt, à titre d'exemples non limitatifs un paramètre hémodynamique ou la fonction des résidus, d'un système dynamique artère/tissu/veine d'une région d'intérêt. On entend par « région d'intérêt » toute région comportant au moins un voxel. Néanmoins, une région d'intérêt ne saurait être limitée à un seul voxel, mais peut comprendre une pluralité de voxels, sélectionnés manuellement ou automatiquement. Selon l'invention, ladite quantité d'intérêt peut être estimée pour chaque voxel au moyen d'un procédé 100 conforme à l'invention tel que décrit précédemment mis en œuvre itérativement pour chaque voxel par les moyens de traitement de l'unité de traitement 4. Il est ainsi possible, selon l'étape 210, d'estimer une quantité d'intérêt sur une pluralité de voxels définissant une région d'intérêt, pouvant s'étendre éventuellement sur plusieurs stations parmi une pluralité de stations, pour par exemple, effectuer l'analyse d'organes dont la taille dépasse le champ d'acquisition de l'appareil d'analyse par imagerie de perfusion.

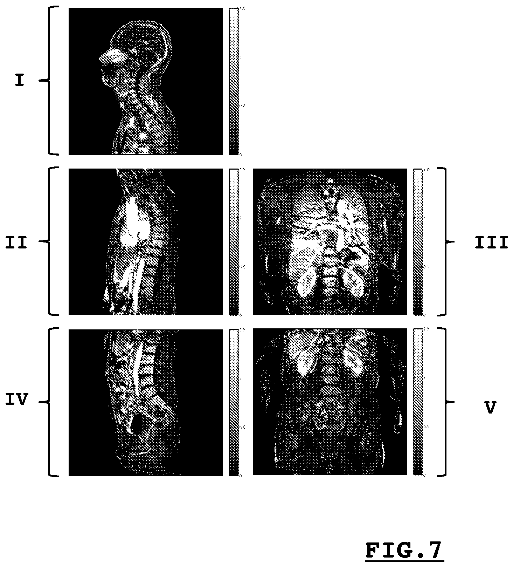

En complément, ledit procédé peut comporter une étape subséquente 211 pour déclencher une restitution de ladite quantité d'intérêt, à savoir un paramètre hémodynamique 14, estimée pour les voxels de la région d'intérêt par lesdits moyens de restitution selon un format approprié. La restitution peut s'apparenter avantageusement à une carte de paramètres où chaque voxel correspond à un degré d'intensité en lien avec la quantité d'intérêt estimée. Une telle étape peut comporter une sous-étape pour visualiser une carte de paramètre pour chaque station. Un tel mode de réalisation est décrit en liaison avec la figure 7. La figure 7 présente cinq cartes relatives au flux sanguin, également connu sous la terminologie $K_{trans}$, estimé à partir d'une pluralité de stations, avantageusement cinq, conformément à l'invention, les cinq cartes correspondant aux stations I à V. De telles cartes pour les stations I à V peuvent être avantageusement exploitées dans le cadre de l'étude du myélome, cancer touchant les cellules de la moelle épinière. Chaque station comporte ainsi une représentation indépendante l'une de l'autre.

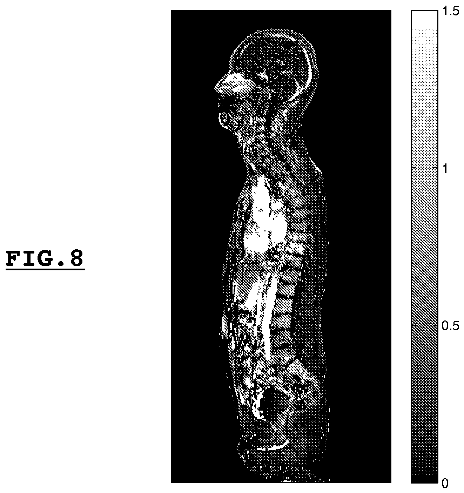

En variante ou en complément, le procédé 200 peut comporter une étape 220 pour générer un volume global à partir d'une pluralité de stations. Il peut en outre comporter une étape pour déclencher la restitution par exemple sous la forme d'une carte consolidée intégrant ou fusionnant les cartes produites pour ladite pluralité de stations. Une telle étape 220 peut ainsi comprendre une sous-étape pour joindre différentes cartes de stations comprises dans la pluralité de stations. Un tel mode de réalisation est décrit en liaison avec la figure 8.

La figure 8 présente une carte consolidée d'une pluralité de stations à partir de cartes de paramètres relatives audit flux sanguin estimé, lesdites cartes étant représentées à titre d'exemples non limitatifs en liaison avec la figure 7. Deux cartes adjacentes parmi les différentes stations I à V, comportent généralement une zone de recouvrement, ladite zone de recouvrement comportant avantageusement des voxels périphériques correspondants, se correspondant deux à deux et formant des paires de voxels correspondants. La quantité d'intérêt en lien avec de telles paires de voxels pourra être estimée par l'unité de traitement 4 comme le résultat d'une combinaison linéaire entre les estimations de ladite quantité d'intérêt des voxels correspondants au sein des deux stations adjacentes. Ainsi, toute discontinuité au sein de la carte consolidée, résultant d'une fusion des cartes de paramètre propres à chaque station, parmi les stations I à V, est prévenue ou atténuée : l'observation de la présence d'une telle discontinuité pourra être vérifiée grâce aux moyens de restitution 5 d'un appareil d'imagerie médicale, tel que celui décrit en liaison avec les figures 1 ou 2. D'après l'exemple décrit en liaison avec la figure 8, les cartes des stations I et II, ainsi que II et IV, sont fusionnées pour générer une carte consolidée.

En variante ou en complément, le procédé 200 peut comporter une étape 230 pour vérifier l'estimation de la quantité d'intérêt estimée : une telle vérification peut être réalisée automatiquement ou visuellement. Ladite étape de vérification 230 peut consister à détecter toute discontinuité notable sur une carte consolidée. En variante ou en complément, une telle étape de vérification 230 peut consister à vérifier automatiquement que les valeurs des estimations de ladite quantité d'intérêt des voxels correspondants sont cohérentes, c'est-à-dire que les valeurs de telles estimations se placent dans une plage de valeurs peu étendues. Une telle vérification peut être réalisée en utilisant des tests statistiques, tels qu'à titre d'exemples non limitatifs, le test de Kolmogorov-Smirnov ou de manière préférée, la théorie bayésienne. Le procédé 200 peut alors comporter une étape 231 pour déclencher une mise en exergue via les moyens de restitution, des voxels jugés en 230 comme peu cohérents.

Grâce aux nouvelles estimations et/ou cartes présentées précédemment, l'invention permet de mettre à la disposition d'un praticien tout un ensemble d'informations pertinentes et cohérentes, informations qui ne pouvaient être disponibles à l'aide des techniques connues de l'état de l'art. Cette mise à disposition est rendue possible par une adaptation de l'unité de traitement 4 selon les figures 1 ou 2 en ce que les moyens de traitement mettent en œuvre un procédé d'estimation d'une quantité d'intérêt comportant la construction d'une fonction d'entrée artérielle à partir de données de perfusion en lien avec une ou plusieurs stations. Une telle mise en œuvre est avantageusement rendue possible par le chargement ou l'enregistrement au sein de moyens de mémorisation coopérant avec lesdits moyens de traitement, d'un produit programme d'ordinateur. Ce dernier comporte en effet des instructions interprétables et/ou exécutables par lesdits moyens de traitement. L'interprétation ou l'exécution desdites instructions déclenche la mise en œuvre d'un procédé 100 ou 200 conforme à l'invention. Les moyens pour communiquer avec le monde extérieur de ladite unité de traitement peuvent délivrer une quantité d'intérêt, à savoir les paramètres estimés 14, selon un format approprié à des moyens de restitution aptes à la restituer à un utilisateur 6, ladite quantité d'intérêt estimée pouvant être avantageusement restituée sous la forme par exemple de cartes telles qu'illustrées par les figures 7 et 8.

Grâce à l'invention, les informations délivrées sont ainsi plus nombreuses, cohérentes, reproductibles et justes. Les informations, dont le praticien dispose, sont ainsi de nature à accroître la confiance du praticien dans sa détermination d'un diagnostic et sa prise de décision.

**Revendications**

1. Procédé (100) pour produire une estimation d'une quantité d'intérêt (14) d'un système dynamique artère/tissu/veine d'un volume élémentaire - dit voxel - d'un organe d'un patient, ledit procédé (100) étant mis en œuvre par des moyens de traitement d'une unité de traitement (4) d'un système d'analyse d'imagerie de perfusion par résonance magnétique, et comportant une étape (130) pour estimer ladite quantité d'intérêt (14) à partir de données de perfusion liées respectivement à une station parmi des données de perfusion liées respectivement à une pluralité de stations à partir de données de perfusion (12) liées à ladite station, chaque station étant un volume correspondant à un champ d'acquisition défini par un appareil d'imagerie médicale par résonance magnétique comprenant une table mobile sur

   laquelle le patient est déplacé de station en station pour l'acquisition desdites données de perfusion, ledit procédé: comporte une étape (120) pour construire une fonction d'entrée artérielle commune à partir de données de perfusion liées à au moins une station parmi ladite pluralité de stations, et ladite étape (130) pour estimer la quantité d'intérêt à partir des données de perfusion liées à une station parmi ladite pluralité exploite, pour toute station de ladite pluralité de stations, la fonction d'entrée artérielle commune construite et les données de perfusion liées à ladite station pour estimer la quantité d'intérêt, ladite station comprenant le volume élémentaire de l'organe du patient.

2. Procédé (100) selon la revendication précédente, pour lequel l'étape (120) pour construire une fonction d'entrée artérielle commune est mise en œuvre à partir des données de perfusion liées à au moins deux stations parmi ladite pluralité de stations.

3. Procédé (100) selon l'une quelconque des revendications précédentes, pour lequel l'étape (120) pour construire une fonction d'entrée artérielle commune comporte une étape pour déterminer une fonction d'entrée artérielle propre à une station parmi ladite au moins une station, la fonction d'entrée artérielle commune étant construite à partir de ladite fonction d'entrée artérielle propre.

4. Procédé (100) selon l'une quelconque des revendications précédentes, le système d'analyse d'imagerie de perfusion par résonance magnétique comportant des moyens de restitution (5) à un utilisateur (6) dudit système, lesdits moyens de restitution (5) coopérant avec l'unité de traitement (4), ledit procédé comportant une étape subséquente (121) pour déclencher une restitution de la fonction d'entrée artérielle commune par lesdits moyens de restitution.

5. Procédé (100) selon l'une quelconque des revendications précédentes, comportant une étape préalable (110) de prétraitement des données de perfusion (12), ladite étape étant agencée pour corriger lesdites données de perfusion (12).

6. Procédé (200) pour produire une estimation d'une quantité d'intérêt d'un système dynamique artère/tissu/veine d'une région d'intérêt, ladite région comportant au moins un voxel, ladite quantité d'intérêt étant estimée par voxel au moyen d'un procédé (100) conforme à l'une quelconque des revendications 1 à 5.

7. Procédé (200) selon la revendication précédente, pour lequel ladite région d'intérêt s'étend sur plusieurs stations parmi la pluralité de stations.

8. Procédé (200) selon l'une quelconque des revendications 6 ou 7, le système d'analyse d'imagerie de perfusion par résonance magnétique comportant des moyens de restitution (5) à un utilisateur (6) dudit système, lesdits moyens de restitution (5) coopérant avec l'unité de traitement (4), ledit procédé comportant une étape (211) subséquente pour déclencher une restitution de ladite quantité d'intérêt estimée pour les voxels de la région d'intérêt par lesdits moyens de restitution (5).

9. Unité de traitement (4) comportant des moyens pour communiquer avec le monde extérieur et des moyens de traitement, coopérant avec des moyens de mémorisation, tel que :

   - les moyens pour communiquer sont adaptés à recevoir du monde extérieur des données de perfusion (12) liées avec une pluralité de stations (I,II,III, IV,V), chaque station étant un volume correspondant à un champ d'acquisition défini par un appareil d'imagerie médicale par résonance magnétique pilotant une table mobile sur laquelle un patient est déplacé de station en station pour l'acquisition desdites données de perfusion ;
   - les moyens de mémorisation comportent des instructions exécutables ou interprétables par les moyens de traitement dont l'interprétation ou l'exécution desdites instructions par lesdits moyens de traitement provoque la mise en œuvre d'un procédé (200) selon l'une quelconque des revendications 6 à 8.

10. Unité de traitement (4) selon la revendication précédente, pour laquelle les moyens pour communiquer sont adaptés à délivrer une quantité d'intérêt estimée (14) selon un format approprié à des moyens de restitution (5) aptes à la restituer à un utilisateur (6).

11. Système d'analyse d'imagerie de perfusion par résonance magnétique comportant une unité de traitement (4) selon l'une quelconque des revendications 9 et 10 et des moyens de restitution (5) adaptés à restituer à un utilisateur (6) une quantité estimée (14) selon un procédé conforme à l'une quelconque des revendications 6 à 8 et mis en œuvre par ladite unité de traitement (4).

12. Produit programme d'ordinateur comportant une ou plusieurs instructions interprétables ou exécutables par les moyens de traitement d'une unité de traitement (4) conforme à l'une quelconque des revendications 9 et 10, ladite unité de traitement (4) comportant en outre des moyens de mémorisation ou coopérant avec de tels moyens de mémorisation, ledit programme étant chargeable dans lesdits moyens de mémorisation, **caractérisé en ce que** l'interprétation ou l'exécution desdites instructions par lesdits moyens de traitement provoque la mise en œuvre d'un procédé (200) selon l'une quelconque des revendications 6 à 8.

**Patentansprüche**

1. Verfahren (100) zum Erzeugen einer Schätzung einer Menge von Interesse (14) eines dynamischen Arterien/Gewebe/Venen-Systems eines Volumenelements - Voxel genannt - eines Organs eines Patienten, wobei das genannte Verfahren (100) von Verarbeitungsmitteln einer Verarbeitungseinheit (4) eines Magnetresonanz-Perfusionsbildgebungs-Analysesystems durchgeführt wird und einen Schritt (130) zum Schätzen der genannten Menge von Interesse (14) auf der Basis von Perfusionsdaten jeweils in Verbindung mit einer Station aus Perfusionsdaten jeweils in Verbindung mit einer Mehrzahl von Stationen, auf der Basis von Perfusionsdaten (12) in Verbindung mit der genannten Station beinhaltet, wobei jede Station ein Volumen ist, das einem Erfassungsfeld entspricht, definiert durch ein medizinisches Magnetresonanz-Bildgebungsgerät, das einen beweglichen Tisch umfasst, auf dem der Patient zum Erfassen der genannten Perfusionsdaten von Station zu Station bewegt wird, wobei das genannte Verfahren: einen Schritt (120) zum Konstruieren einer gemeinsamen arteriellen Eingangsfunktion auf der Basis von Perfusionsdaten in Verbindung mit mindestens einer Station aus der genannten Mehrzahl von Stationen beinhaltet, und der genannte Schritt (130) zum Schätzen der Menge von Interesse auf der Basis der Perfusionsdaten in Verbindung mit einer Station aus der genannten Mehrzahl, für jede Station der genannten Mehrzahl von Stationen die konstruierte gemeinsame arterielle Eingangsfunktion und die Perfusionsdaten in Verbindung mit der genannten Station zum Schätzen der Menge von Interesse nutzt, wobei die genannte Station das Volumenelement des Organs des Patienten umfasst.

2. Verfahren (100) nach dem vorherigen Anspruch, bei dem der Schritt (120) zum Konstruieren einer gemeinsamen arteriellen Eingangsfunktion auf der Basis von Perfusionsdaten in Verbindung mit mindestens zwei Stationen aus der genannten Mehrzahl von Stationen durchgeführt wird.

3. Verfahren (100) nach einem der vorherigen Ansprüche, bei dem der Schritt (120) zum Konstruieren einer gemeinsamen arteriellen Eingangsfunktion einen Schritt zum Bestimmen einer eigenen arteriellen Eingangsfunktion für eine Station aus der genannten mindestens einen Station beinhaltet, wobei die gemeinsame arterielle Eingangsfunktion auf der Basis der genannten eigenen arteriellen Eingangsfunktion konstruiert wird.

4. Verfahren (100) nach einem der vorherigen Ansprüche, wobei das Magnetresonanz-Perfusionsbildgebungs-Analysesystem Mittel (5) zur Wiedergabe an einen Benutzer (6) des genannten Systems umfasst, wobei die genannten Wiedergabemittel (5) mit der Verarbeitungseinheit (4) zusammenarbeiten, wobei das genannte Verfahren einen nachfolgenden Schritt (121) zum Auslösen einer Wiedergabe der gemeinsamen arteriellen Eingangsfunktion durch die genannten Wiedergabemittel beinhaltet.

5. Verfahren (100) nach einem der vorherigen Ansprüche, das einen Vorabschritt (110) des Vorverarbeitens der Perfusionsdaten (12) beinhaltet, wobei der genannte Schritt zum Korrigieren der genannten Perfusionsdaten (12) eingerichtet ist.

6. Verfahren (200) zum Erzeugen einer Schätzung einer Menge von Interesse eines dynamischen Arterien/Gewebe/Venen-Systems einer Region von Interesse, wobei die genannte Region mindestens ein Voxel umfasst, wobei die genannte Menge von Interesse pro Voxel gemäß einem Verfahren (100) gemäß einem der Ansprüche 1 bis 5 geschätzt wird.

7. Verfahren (200) nach dem vorherigen Anspruch, bei dem sich die genannte Region von Interesse über mehrere Stationen aus der Mehrzahl von Stationen erstreckt.

8. Verfahren (200) nach Anspruch 6 oder 7, wobei das Magnetresonanz-Perfusionsbildgebungs-Analysesystem Mittel (5) zur Wiedergabe an einen Benutzer (6) des genannten Systems umfasst, wobei die genannten Wiedergabemittel (5) mit der Verarbeitungseinheit (4) zusammenarbeiten, wobei das genannte Verfahren einen nachfolgenden Schritt (211) zum Auslösen einer Wiedergabe der genannten geschätzten Menge von Interesse für die Voxel der Region von Interesse durch die genannten Wiedergabemittel (5) beinhaltet.

9. Verarbeitungseinheit (4), die Mittel zur Kommunikation mit der Außenwelt und Verarbeitungsmittel umfasst, die mit Speichermitteln zusammenarbeiten, so dass:

   - die Mittel zur Kommunikation zum Empfangen, von der Außenwelt, von Perfusionsdaten (12) in Verbindung mit einer Mehrzahl von Stationen (I,II,III,IV,V) ausgelegt sind, wobei jede Station ein Volumen ist, das einem Erfassungsfeld entspricht, definiert durch ein medizinisches Magnetresonanz-Bildgebungsgerät, das einen beweglichen Tisch steuert, auf dem ein Patient zum Erfassen der genannten Perfusionsdaten von Station zu Station bewegt wird;
- die Speichermittel Befehle enthalten, die von den Verarbeitungsmitteln ausgeführt oder interpretiert werden können, wobei die Interpretation oder Ausführung der genannten Befehle durch die genannten Verarbeitungsmittel die Durchführung eines Verfahrens (200) nach einem der Ansprüche 6 bis 8 bewirkt.

10. Verarbeitungseinheit (4) nach dem vorherigen Anspruch, bei der die Mittel zur Kommunikation zum Liefern einer geschätzten Menge von Interesse (14) in einem geeigneten Format an Wiedergabemittel (5) ausgelegt sind, die zu ihrer Wiedergabe an einen Benutzer (6) geeignet sind.

11. Magnetresonanz-Perfusionsbildgebungs-Analysesystem, das eine Verarbeitungseinheit (4) nach einem der Ansprüche 9 und 10 und Wiedergabemittel (5) umfasst, die zum Wiedergeben einer geschätzten Menge (14) gemäß einem Verfahren an einen Benutzer (6) ausgelegt sind, das einem der Ansprüche 6 bis 8 entspricht und von der genannten Verarbeitungseinheit (4) durchgeführt wird.

12. Computerprogrammprodukt mit einem oder mehreren Befehlen, die von den Verarbeitungsmitteln einer Verarbeitungseinheit (4) gemäß einem der Ansprüche 9 und 10 interpretiert oder ausgeführt werden können, wobei die genannte Verarbeitungseinheit (4) außerdem Speichermittel umfasst oder mit solchen Speichermitteln zusammenarbeitet, wobei das genannte Programm in die genannten Speichermittel geladen werden kann, **dadurch gekennzeichnet, dass** die Interpretation oder Ausführung der genannten Befehle durch die genannten Verarbeitungsmittel die Durchführung eines Verfahrens (200) nach einem der Ansprüche 6 bis 8 bewirkt.

**Claims**

1. Method (100) for producing an estimate of a quantity of interest (14) of a dynamic artery/tissue/vein system of an elementary volume - called voxel - of an organ of a patient, said method (100) being implemented by the processing means of a processing unit (4) of a perfusion-weighted magnetic resonance imaging analysis system, and comprising a step (130) for estimating said quantity of interest (14) based on perfusion data respectively associated with one station from among the perfusion data respectively associated with a plurality of stations based on perfusion data (12) associated with said station, each station being a volume corresponding to an acquisition field defined by a magnetic resonance medical imaging device, comprising a mobile table on which the patient is moved from station to station to acquire said perfusion data, said method:
comprises a step (120) for constructing a common arterial input function based on perfusion data associated with at least one station from among said plurality of stations, and said step (130) for estimating the quantity of interest based on the perfusion data associated with one station from among said plurality uses, for every station of said plurality of stations, the constructed common arterial input function and the perfusion data associated with said station to estimate the quantity of interest, said station comprising the elementary volume of the organ of the patient.

2. Method (100) according to the preceding claim, in which the step (120) for constructing a common arterial input function is implemented based on perfusion data associated with at least two stations from among said plurality of stations.

3. Method (100) according to any one of the preceding claims, in which the step (120) for constructing a common arterial input function comprises a step for determining an arterial input function dedicated to one station from among said at least one station, the common arterial input function being constructed based on said dedicated arterial input function.

4. Method (100) according to any one of the preceding claims, the perfusion-weighted magnetic resonance imaging analysis system comprising output means (5) for a user (6) of said system, said output means (5) cooperating with the processing unit (4), said method comprising a subsequent step (121) for triggering an output of the common arterial input function by said output means.

5. Method (100) according to any one of the preceding claims, comprising a preliminary step (110) of pre-processing the perfusion data (12), said step being arranged to correct said perfusion data (12).

6. Method (200) for producing an estimate of a quantity of interest of a dynamic artery/tissue/vein system of a region of interest, said region comprising at least one voxel, said quantity of interest being estimated per voxel by means of a method (100) according to any one of claims 1 to 5.

7. Method (200) according to the preceding claim, in which said region of interest extends over several stations from among the plurality of stations.

8. Method (200) according to either of claims 6 or 7, the perfusion-weighted magnetic resonance imaging analysis system comprising output means (5) for a user (6) of said system, said output means (5) cooperating with the processing unit (4), said method comprising a subsequent step (211) for triggering an output of said estimated quantity of interest for the voxels of the region of interest by said output means (5).

9. Processing unit (4) comprising communication means with the outside world and processing means, cooperating with storage means, such that:

    - the communication means are suitable for receiving from the outside world perfusion data (12) associated with a plurality of stations (I,II,III,IV,V), each station being a volume corresponding to an acquisition field defined by a magnetic resonance medical imaging device controlling a mobile table on which a patient is moved from station to station to acquire said perfusion data;
    - the storage means comprise instructions that can be executed or interpreted by the processing means, wherein the interpretation or execution of said instructions by said processing means causes the implementation of a method (200) according to any one of claims 6 to 8.

10. Processing unit (4) according to the preceding claim, in which the communication means are suitable for delivering an estimated quantity of interest (14) in a suitable format to output means (5) capable of outputting it to a user (6).

11. Perfusion-weighted magnetic resonance imaging analysis system comprising a processing unit (4) according to either of claims 9 and 10 and output means (5) suitable for outputting an estimated quantity (14) to a user (6) according to a method according to any one of claims 6 to 8 and implemented by said processing unit (4).

12. Computer program product comprising one or more instructions that can be interpreted or executed by the processing means of a processing unit (4) according to either of claims 9 and 10, said processing unit (4) further comprising storage means or cooperating with such storage means, said program being able to be loaded in said storage means, **characterized in that** the interpretation or execution of said instructions by said processing means causes the implementation of a method (200) according to any one of claims 6 to 8.

EP 3 207 397 B1

**FIG.1**

**FIG.2**

17

**FIG.3**

**FIG.4**

100

110

120 → 121

130 → 131

**FIG.5A**

200

210 → 211

220 → 221

230 → 231

**FIG.5B**

$C_a(t)$

C(t)

I ⟷ ●

II ⟷ ▲

III ⟷ ◆

IV ⟷ ⬟

**FIG.6**

0    60    120    180    240    300    t (sec)

**FIG.7**

**FIG.8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20110110488 A1

**Littérature non-brevet citée dans la description**

- **LUCIANI A. et al.** Hématologie : applications IRM corps entier. *Journal de Radiologie,* 2009, vol. 90 (10), 1338

- **DE LIN S. et al.** IRM corps entier en haute resolution avec injection dynamique de gadolinium dans les syndromes lymphoproliferatifs. *Journal de Radiologie,* 2007, vol. 88 (10), 1445